# EUROPEAN PATENT APPLICATION

(11) **EP 4 579 667 A1**
(43) Date of publication of application: **02.07.2025**
(21) Application number: 24221062.3
(22) Date of filing: 18.12.2024
(51) Int. Cl.: G16B 5/20, G16B 20/50, G16B 35/20

(54) **METHOD FOR EXTENDING PEPTIDE LINKERS BETWEEN PEPTIDES**

(30) Priority: 27.12.2023 US 202363615272 P; 21.11.2024 TW 113144875
(71) Applicant: Acer Incorporated, New Taipei City 221 (TW)
(72) Inventor: TU, Chih-Wei, 221 New Taipei City (TW); CHAN, Yun-Hsuan, 221 New Taipei City (TW); PAN, Ke-Han, 221 New Taipei City (TW); TSAI, Tsung-Hsien, 221 New Taipei City (TW)
(74) Representative: Haseltine Lake Kempner LLP

(57) **Abstract**

Provided is a method for extending peptide linkers between peptides, including the following steps. All combinations of the peptide linkers of a length of n are listed, and a combination of a best peptide linker is generated. After that, a first condition and a second condition are considered to extend the peptide linkers. The first condition is that a cutting point of the peptide linkers is in the middle, and a center of extending the peptide linkers is selected from amino acids of a cutting point of the best peptide linker. The second condition is that amino acids of a N-terminal and amino acids of a C-terminal of the peptide linkers are searched, and the amino acids of the N-terminal and the amino acids of the C-terminal have a higher probability of being cut off.

## Description

### BACKGROUND

### Technical Field

This disclosure relates to a method for extending peptide linkers, and in particular, to a method for extending peptide linkers between peptides.

### Description of Related Art

With the advancement of next-generation gene sequencing and artificial intelligence, personalized cancer vaccines have emerged as one of the modalities for cancer treatment. The production and manufacturing process of personalized cancer vaccines encompasses the following: prioritization and selection of tumor neoantigens, followed by the utilization of peptide linkers to concatenate high-priority peptides, thereby facilitating subsequent production, manufacturing, and vaccine administration. Peptide linkers not only serve to connect various potential tumor neoantigens but also play an auxiliary role in enhancing the likelihood of peptide incorporation into the immune response mechanism.

Considering the cost of synthesizing peptide linkers and their efficacy, the optimal length of peptide linkers remains a subject of debate. The current viable approach involves employing combinatorial methods to explore and validate various lengths and amino acid combinations. However, this methodology results in an exponential increase in time and computational costs associated with identifying the optimal peptide linkers, correlating with the length of the linker and the number of peptides to be linked. Consequently, there is a need to develop a method for extending peptide linkers between peptides that reduces time and computational costs while maintaining the efficacy of the peptide linkers.

### SUMMARY

The disclosure provides a method for extending peptide linkers between peptides, capable of reducing time and computational costs while maintaining the efficacy of the peptide linkers.

A method for extending peptide linkers between peptides of the disclosure includes the following. All combinations of the peptide linkers of a length of n are listed, and a combination of a best peptide linker is generated. After that, a first condition and a second condition are considered to extend the peptide linkers. The first condition is that a cutting point of the peptide linkers is in the middle, and a center of extending the peptide linkers is selected from amino acids of a cutting point of the best peptide linker. The second condition is that amino acids of a N-terminal and amino acids of a C-terminal of the peptide linkers are searched, and the amino acids of the N-terminal and the amino acids of the C-terminal have a higher probability of being cut off.

In an embodiment of the disclosure, when a length of an extended peptide linker is an even number, two center positions exit.

In an embodiment of the disclosure, in the second condition, P11 is peptide amino acids connected to the N-terminal of the peptide linkers, L1 is the amino acids of the N-terminal of the peptide linkers, and P11 and L1 satisfy the following formula:
Prob(P11)-Prob(L1)>0.5,
where Prob(x) represents probability of being cut off at a x position.

In an embodiment of the disclosure, in the second condition, L1 is the amino acids of the N-terminal of the peptide linkers, L2 is middle amino acids of the peptide linkers, and L1 and L2 satisfy the following formula:
Prob(L2)-Prob(L1)>0.5,
where Prob(x) represents probability of being cut off at a x position.

In an embodiment of the disclosure, in the second condition, L2 is middle amino acids of the peptide linkers, L3 is the amino acids of the C-terminal of the peptide linkers, and L2 and L3 satisfy the following formula:
Prob(L2)-Prob(L3)>0.5,
where Prob(x) represents probability of being cut off at a x position.

Based on the above, the disclosure provides a method for extending peptide linkers between peptides by adopting the concept of greedy algorithm, retaining the properties of the best peptide linkers in the current state, and reducing the range of amino acid searches to extend the peptide linkers, which therefore effectively reduces unnecessary operations, thereby reducing time and operation costs while maintaining the effectiveness of the peptide linker.

To make the aforementioned more comprehensible, several embodiments accompanied with drawings are described in detail as follows.

### BRIEF DESCRIPTION OF THE DRAWINGS

The accompanying drawings are included to provide a further understanding of the disclosure, and are incorporated in and constitute a part of this specification. The drawings illustrate example embodiments of the disclosure and, together with the description, serve to explain the principles of the disclosure.
FIG. 1 is a flow chart of a method for extending peptide linkers between peptides according to an embodiment of the disclosure.
FIG. 2, FIG. 3, and FIG. 4 are schematic diagrams of a method for considering A first condition according to an embodiment of the disclosure.
FIG. 5 and FIG. 6 are schematic diagrams of a method for considering a second condition according to an embodiment of the disclosure.
FIG. 7 and FIG. 8 are schematic diagrams considering the second condition according to an embodiment of the disclosure.

### DESCRIPTION OF THE EMBODIMENTS

Embodiments are enumerated below and described in detail with reference to the drawings. However, the embodiments provided are not intended to limit the scope of the disclosure. In addition, the terms "including", "comprising", "having", etc. used in the article are all open terms, which means "including but not limited to".

In the drawings of the disclosure, the solid position is the point that is expected to be cut off, while the hollow position indicates the opposite.

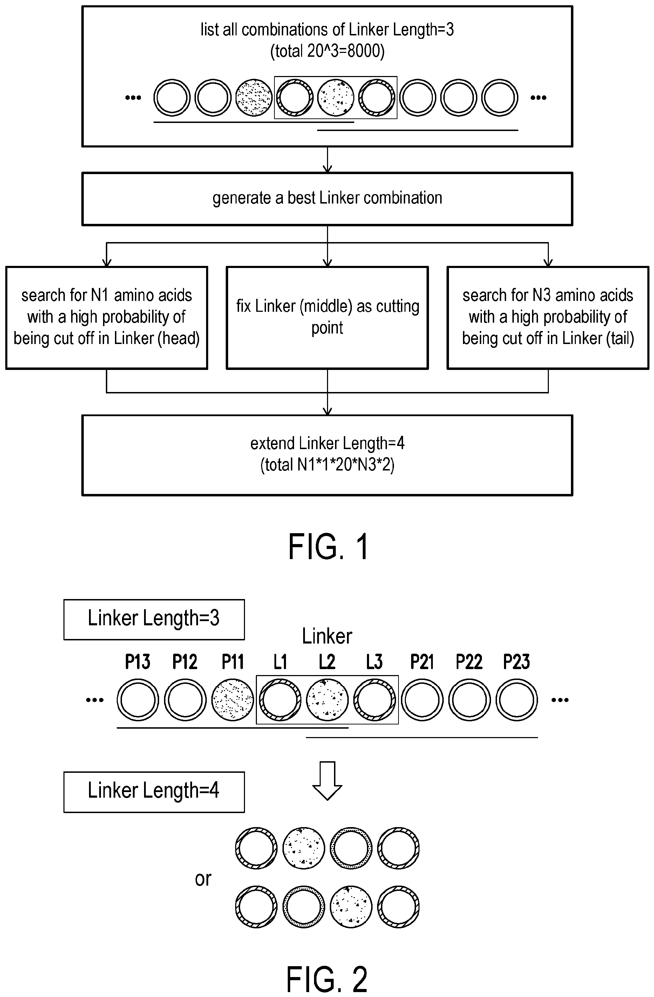

FIG. 1 is a flow chart of a method for extending peptide linkers between peptides according to an embodiment of the disclosure.

Please refer to FIG. 1. In the method for extending peptide linkers between peptides in the disclosure, all combinations of peptide linkers of length of n are listed first, and there are a total of 20 common amino acids, so that there is a combination of 20^n. In this embodiment, n is, for example, 3, that is, all combinations of peptide linkers of length of 3 are listed, but the disclosure is not limited thereto, and can be adjusted to different peptide linker lengths according to actual needs.

After that, continuing referring to FIG. 1, a combination of a best peptide linker is generated. Although extending the peptide linker increases the number of search combinations, the disclosure retains the properties of the best peptide linker in its current state, and extends the peptide linker by narrowing the scope of the amino acid search to find the combination of the best peptide linker after the extending. The method for extending peptide linkers between peptides of the disclosure is mainly in that when the length of the peptide linker is extended, the scope of search can be narrowed to reduce the cost of computing time. In this embodiment, based on the best peptide linker with a peptide linker length of 3, the peptide linker combination is extended outwardly. When extending, a first condition and a second condition are considered to extend the peptide linker, which is described in detail below with reference to the drawings for the first and second conditions and the second condition, respectively.

FIG. 2, FIG. 3, and FIG. 4 are schematic diagrams of a method for considering a first condition according to an embodiment of the disclosure.

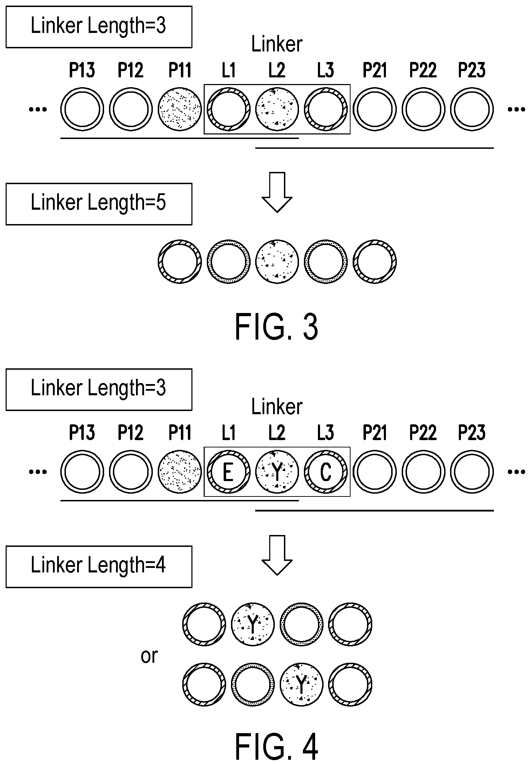

Please refer to FIG. 2 and FIG. 3 first. The first condition is that a cutting point of the peptide linker is in the middle, and the center of the extended peptide linker is selected from the amino acid of the cutting point of the best peptide linker of length of 3. If the length of the extended peptide linker is an even number, there are two center positions. In more detail, the first condition is that the cutting point of the peptide linker is located at the central position of the peptide linker sequence. When the length of the peptide linker is an odd number (for example, 3), the central position is the amino acid in the middle of the sequence. When the length of the peptide linker is an even number, the central position refers to the position between two adjacent amino acids in the middle of the sequence. L1, L2, and L3 are the positions of the peptide linker, P1k is the peptide connected to the N-terminal of the peptide linker, and P2k is the peptide connected to the C-terminal of the peptide linker, where k represents the distance from the peptide linker. Assuming that the cutting point of the best peptide linker of length of 3 is L2, the center point is fixed to the amino acid of L2. Please refer to FIG. 4. For example, if the best peptide linker of length of 3 is EYC and the cutting point is Y, the center point of the best peptide linker of length of 4 is only considered to be Y.

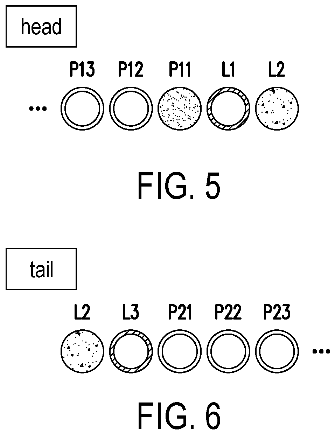

FIG. 5 and FIG. 6 are schematic diagrams of a method for considering a second condition according to an embodiment of the disclosure. Based on the fact that the head and tail of the peptide linker can affect the ease with which the peptide itself can be cut, the second condition is to search for the N-terminal amino acid and C-terminal amino acid of the peptide linker. The N-terminal amino acid and C-terminal amino acid exhibit a higher probability of being cut off.

Please refer to FIG. 5. The amino acid conditions for extending the N-terminal (head) of the peptide linker include the following three points.

The first point is the relationship between peptide linker and peptide. P11 is the C-terminal amino acid of the peptide connected to the N-terminal of the peptide linker, and L1 is the N-terminal amino acid of the peptide linker, which satisfy the following formula:
Prob(P11)-Prob(L1)>0.5 ,
where Prob(x) represents the probability of being cut off at the x position.

The second point is the relationship between peptide linkers. L1 is the N-terminal amino acid of the peptide linker, and L2 is the middle amino acid of the peptide linker, which satisfy the following formula:
Prob(L2)-Prob(L1)>0.5 ,
where Prob(x) represents the probability of being cut off at the x position.

The third point is the intersection of the two relationships. If there is no intersection, use the following distance formula to sort from small to large and then take TopN, but not limited to P13, can also be to P1k:
|[0,0,1,0,1]-[Prob(P13),Prob(P12),Prob(P11),Prob(L1),Prob(L2)]|.

Please refer to FIG. 6. The amino acid conditions for extending the C-terminal (tail) of the peptide linker include the following three points.

The first point is the relationship between the peptide linker and the peptide, for example, it satisfies the following formula:
all(Prob(L3),Prob(21),Prob(22),Prob(23))<0.5

The second point is the relationship between peptide linkers. L2 is the middle amino acid of the peptide linker, and L3 is the C-terminal amino acid of the peptide linker, which satisfy the following formula:
Prob(L2)-Prob(L3)>0.5 ,
where Prob(x) represents the probability of being cut off at the x position.

The third point is the intersection of the two relationships. If there is no intersection, use the following distance formula to sort from small to large and then take TopN, but not limited to P23, can also be to P2k:
|[1,0,0,0,0]-[Prob(L2),Prob(L3),Prob(P21),Prob(P22),Prob(P23)]|.

In this embodiment, multiple methods can be integrated, using Pepsickle or NetCleave, to equalize the number of methods, thereby increasing the quantity of peptide linkers through similarity, consequently generating amino acids with a high probability of being cut off.

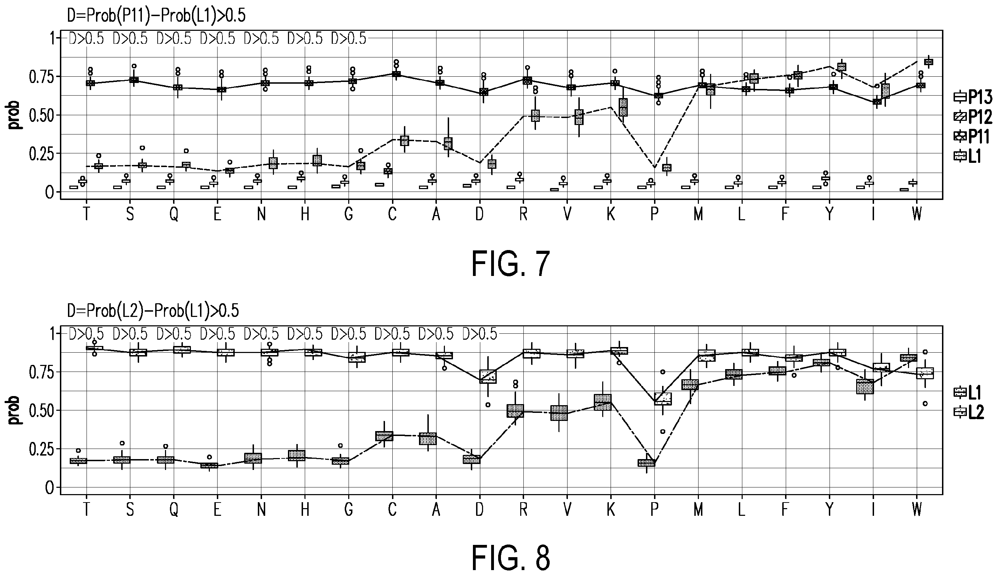

FIG. 7 and FIG. 8 are schematic diagrams considering the second condition according to an embodiment of the disclosure.

Please refer to FIG. 7. In this example, the N-terminal (head) of the peptide linker is used as an example to explore the relationship between the peptide linker and the peptide. There are 8 amino acids that meet the conditions (T, S, Q, E, N, H, G, C). Please refer to FIG. 8 to explore the relationship between peptide linkers, and there are 10 amino acids that meet the conditions (T, S, Q, E, N, H, G, C, A, D). Integrating the intersection of the above results, the amino acid candidates are T, S, Q, E, N, H, G, C, narrowing the number of amino acids from 20 to 8.

In this way, compared to the prior art, if the length of the peptide linker is set to 3, there are 20 amino acid possibilities at each position, so a peptide linker with a length of 3 will generate 8000 combinations. If the length of the peptide linker is set to 4, then 160,000 combinations will be generated, and so on. In addition to the exponential increase in time and calculation, the calculation of all peptide linkers of length of 4 will count peptide linkers that are already poorly characterized at length of 3, resulting in wasted calculations and a reduction in the effectiveness of the peptide linkers. In comparison, as shown in Table 1 below, the method of extending peptide linkers between peptides of the present disclosure reduces time by 79% compared to the prior art.

**Table 1**

| **Method** | **Length of peptide linker** | **Number of peptide linker combinations** | **Pepsickle time** | **NetCleave time** |
|---|---|---|---|---|
| Prior art | 3 | 8000 | 4.7 mins | 6.2 mins |
| | 4 | 160000 | 60 mins | 96 mins |
| Present disclosure | 4 | far less than 160000 | 13 mins | 21 mins |

To sum up, the disclosure provides a method for extending peptide linkers between peptides by adopting the concept of greedy algorithm, retaining the properties of the best peptide linkers in the current state, and reducing the range of amino acid searches to extend the peptide linkers. In more detail, the first and second conditions are considered to extend the peptide linker, with the first condition being that the peptide linker is cut in the middle, and the second condition being that the head and tail of the peptide linker affects the ease with which the peptide itself can be cut. Therefore, the method for extending peptide linkers between peptides of the disclosure can effectively reduce unnecessary operations, thereby reducing time and operation costs while maintaining the effectiveness of the peptide linker.

## Claims

1. A method for extending peptide linkers between peptides, comprising:
listing all combinations of the peptide linkers of a length of n;
generating a combination of a best peptide linker; and
considering a first condition and a second condition to extend the peptide linkers,
wherein the first condition is that a cutting point of the peptide linkers is in the middle, and a center of extending the peptide linkers is selected from amino acids of a cutting point of the best peptide linker,
the second condition is that amino acids of a N-terminal and amino acids of a C-terminal of the peptide linkers are searched, and the amino acids of the N-terminal and the amino acids of the C-terminal have a higher probability of being cut off.

2. The method for extending peptide linkers between peptides according to claim 1, wherein when a length of an extended peptide linker is an even number, two center positions exit.

3. The method for extending peptide linkers between peptides according to claim 1, wherein in the second condition, P11 is peptide amino acids connected to the N-terminal of the peptide linkers, L1 is the amino acids of the N-terminal of the peptide linkers, and P11 and L1 satisfy the following formula:
Prob(P11)-Prob(L1)>0.5,
where Prob(x) represents probability of being cut off at a x position.

4. The method for extending peptide linkers between peptides according to claim 1, wherein in the second condition, L1 is the amino acids of the N-terminal of the peptide linkers, L2 is middle amino acids of the peptide linkers, and L1 and L2 satisfy the following formula:
Prob(L2)-Prob(L1)>0.5,
where Prob(x) represents probability of being cut off at a x position.

5. The method for extending peptide linkers between peptides according to claim 1, wherein in the second condition, L2 is middle amino acids of the peptide linkers, L3 is the amino acids of the C-terminal of the peptide linkers, and L2 and L3 satisfy the following formula:
Prob(L2)-Prob(L3)>0.5,
where Prob(x) represents probability of being cut off at a x position.
